# EUROPEAN PATENT APPLICATION

(11) **EP 4 800 707 A1**
(43) Date of publication of application: **02.09.2026**
(21) Application number: 25305266.6
(22) Date of filing: 27.02.2025
(51) Int. Cl.: G16H 50/20, G16H 50/30, G16H 50/70

(54) **COMPUTING SCORE REPRESENTING PERCENTAGE OF OCCURRENCE OF CLINICAL EVENT**

(71) Applicant: Dassault Systèmes, 78140 Vélizy-Villacoublay (FR)
(72) Inventor: COTTIN, Aziliz, 78140 Vélizy-Villacoublay (FR); ZULIAN, Marine, 06560 Valbonne (FR)
(74) Representative: Bandpay & Greuter

(57) **Abstract**

The disclosure notably relates to a computer-implemented method for computing a score representing a percentage of occurrence of a clinical event. The method comprises obtaining a database including a list of P characteristics within a vector *X*, associated with the clinical event. The method comprises obtaining, for a given patient, the modalities corresponding to a subset of the characteristics in the list (*p* ∈ *X**, wherein *X** ⊂ *X*)*.* The method comprises extracting, from the database, the modality weight associated with each modality obtained for the patient. The method comprises computing the score (*scoreₚₐₜᵢₑₙₜ*) for the given patient by summing the extracted modalities weights, using the formula *scoreₚₐₜᵢₑₙₜ* = Σ_{*pj* ∈}*_{X*} α*(*pⱼ* )*.* The method comprises computing a dispersion range associated with the computed score. Such a method forms an improved solution for computing a score representing a percentage of occurrence of a clinical event for a patient.

## Description

### TECHNICAL FIELD

The disclosure relates to the field of biostatistics, and more specifically to methods, system and program for computing a score representing a percentage of occurrence of a clinical event.

### BACKGROUND

In a clinical setting, it is aimed to provide a prediction of a probability of a risk score of occurrence of clinical events related to a disease (for example relapse or death), conditionally to potential risk factors (i.e., "covariates"). These covariates are baseline (i.e., measured at the beginning of a patient's follow-up period).

The example of colon cancer is now presented, but all this can be transposed to the entire field of oncology.

Traditionally, patient's prognostic is evaluated based on the American Joint Committee on Cancer (AJCC) staging classification for colon cancer [1, 2]. The AJCC staging system with the Tumor Node Metastasis (TNM) classification is based on three features: T for primary Tumor, N for regional lymph Nodes, M for distant Metastases. T class is an ordinal classification based on tumor size, ranging from T0 (no evidence of primary tumor) to T4 (e.g., for colon cancer: tumor invades the visceral peritoneum or invades or adheres to adjacent organ or structure). N class is an ordinal classification based on the number of involved lymph nodes, ranging from N0 (no regional lymph node metastasis) to N2 (e.g., for colon cancer: metastasis in four or more lymph nodes). M class is an ordinal classification based on the presence of distant metastases, ranging from M0 (no distant metastasis by imaging or other studies, no evidence of tumor in distant sites or organs) to M1 (metastasis detected to one or more distant sites or organs). The T, N, and M classes are used independently or in combination to determine the cancer stage. This classification is not specific to colon cancer and has been created for the majority of solid cancers. It has been the main prognostic assessment tool for several decades [3, 4]. However, predicting patients' prognosis based on the AJCC staging only has been questioned these last ten years, in particular for its limited ability to account for tumor heterogeneity and its lack of hierarchy in the establishment of the stage [5, 6].

Modified cancer staging has been then proposed, e.g. with the creation of modified TNM classifications (such as in [7]). In the example of colon cancer, there is a tendency towards the desire to integrate certain specific characteristics with greater predictive power, such as the lymph node ratio instead of the number of positive lymph nodes [8], the log odds of positive lymph nodes [9], tumor deposits [10, 11], mismatch repair status (MMR) [12] and microsatellite instability (MSI) [13], or more recently circulating tumor DNA [14, 15].

Following this shift in direction towards new markers of cancer progression, numerous studies investigated whether adding new factors could improve the precision of patients' prognostic. Examples of new biomarkers, among others, in colon cancer include the following. A first example is a persistent elevated postoperative level of CEA. This biomarker is the reference biomarker for establishing the risk of cancer recurrence after surgery [19, 20] and is now part of the European Society for Medical Oncology (ESMO) guidelines. A second example is the MMR status. This biomarker is recommended to be tested from the Institut National du Cancer (INCa) in France at the time of the diagnostic. This biomarker is recommended to be tested for the initiation of targeted therapies and is an accurate predictor of treatment response (for chemotherapy and immunotherapy) [26, 27, 28]. Third examples are the BRAF and RAS (KRAS, nRAS) gene mutations. They are recommended to be tested for the initiation of targeted therapies [26, 29]. Among these new biomarkers, some have been clinically validated and are consensually used today in clinical practice at the time of the diagnostic.

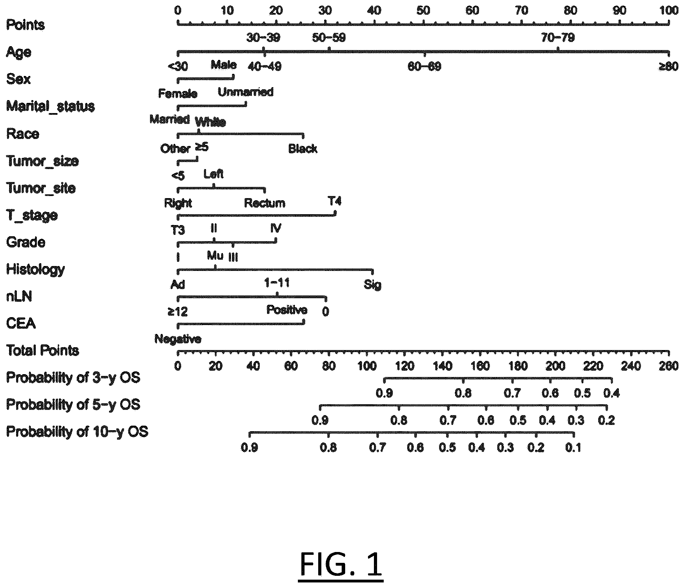

For combining the TNM staging classification with these new markers and create systems that can guide prognosis, the standard approach is based on statistical models (i.e., survival models [16], with in particular the Cox proportional hazard - P.H. - model [17]) and the use of Cox P.H.-based nomograms [18] (i.e., risk scores) based on the clinical, clinic-pathological and biological features that are routinely measured during the patients' care. An example of a nomogram constructed for stage II colon cancer is illustrated in FIG. 1 [30]. When appropriately validated, the nomogram-based approach to estimate a risk score, based on both the TNM classification and new biomarkers, has the potential to personalize the prognostic information available for individual patients and to provide more accurate risks estimations.

Machine learning (ML) and deep learning (DL) algorithms have also emerged in the last fifteen years in this field to integrate these data into predictive algorithms. One of the advantages of these approaches is their ability to handle large number of characteristics and interactions between these characteristics, to fit highly non-linear patterns in the data and automatically model specific types of data (e.g. genomic, transcriptomic, imaging or sequencing data) [22]. They allow to obtain more precise predictions of patient risks compared to statistical models thanks to the integration of these new data.

Building a score based on large predictive algorithms (e.g., a deep learning algorithm that integrates genomic data) requires collecting all of this data on the patients. However, mains limitations are that this information is not always collected in all of the care centers. For some tests, there are very long delays in results (for example several weeks for genetic tests). In some cases, the test results are uninterpretable and must be carried out again (e.g., approximately ^{~}10% of genetic tests are uninterpretable for example, due to the small quantity of DNA extracted or the poor quality that does not allow amplification), which further lengthens the delays in results.

Within this context, there is still a need for an improved solution for computing a score representing a percentage of occurrence of a clinical event for a patient.

### CITED REFERENCES

[1] Stephen B Edge, American Cancer Society American Joint Committee on Cancer, et al. AJCC cancer staging handbook: from the AJCC cancer staging manual, volume 19. Springer, 2010.
[2] Martin R Weiser. Ajcc 8th edition: colorectal cancer. Annals of surgical oncology, 25 :1454-1455, 2018.
[3] Martin R Weiser, Mithat Goenen, Joanne F Chou, Michael W Kattan, and Deborah Schrag. Predicting survival after curative colectomy for cancer: individualizing colon cancer staging. Journal of Clinical Oncology, 29(36) :4796-4802, 2011.
[4] JM Phelip, L Benhaim, O Bouché, N Christou, G Desolneux, and A Dupré. Thésaurus national de cancérologie digestive. Cancer colorectal métastatique [French], 2022.
[5] Alyson L Mahar, Carolyn Compton, Susan Halabi, Kenneth R Hess, Martin RWeiser, and Patti A Groome. Personalizing prognosis in colorectal cancer: A systematic review of the quality and nature of clinical prognostic tools for survival outcomes. Journal of surgical oncology, 116(8) :969-982, 2017.
[6] Neal Bhutiani, Chung-Yuan Hu, Bryan E Palis, Joseph Cotler, Qian Shi, Richard M Goldberg, Mary Kay Washington, Heidi Nelson, Scott Steele, and George J Chang. Lack of hierarchical survival prognosis in ajcc staging for colon cancer: Implications for future summary stage classification., 2023.
[7] Chundong Zhang, Zubing Mei, Junpeng Pei, Masanobu Abe, Xiantao Zeng, Qiao Huang, Kazuhiro Nishiyama, Naohiko Akimoto, Koichiro Haruki, Hongmei Nan, et al. A modified tumor-node-metastasis classification for primary operable colorectal cancer. JNCI Cancer Spectrum, 5(1): pkaa093, 2021.
[8] Yan Yang, Yawei Wang, and Zhengbin Wang. Construction of a new clinical staging system for colorectal cancer based on the lymph node ratio: A validation study. Frontiers in Surgery, 9 :929576, 2022.
[9] Shimpei Ogawa, Michio Itabashi, Yoshiko Bamba, Masakazu Yamamoto, and Kenichi Sugihara. Superior prognosis stratification for stage iii colon cancer using log odds of positive lymph nodes (Iodds) compared to tnm stage classification: the japanese study group for postoperative follow-up of colorectal cancer. Oncotarget, 11(33) :3144, 2020.
[10] Jean-François Delattre, Ayse Selcen Oguz Erdogan, Romain Cohen, Qian Shi, Jean-François Emile, Julien Taieb, Josep Tabernero, Thierry André, Jeffrey A Meyerhardt, Iris D Nagtegaal, et al. A comprehensive overview of tumour deposits in colorectal cancer: Towards a next tnm classification. Cancer treatment reviews, 103 :102325, 2022.
[11] Hideki Ueno, Iris D Nagtegaal, Philip Quirke, Kenichi Sugihara, and Yoichi Ajioka. Tumor deposits in colorectal cancer: Refining their definition in the tnm system. Annals of Gastroenterological Surgery, 7 (2) :225-235, 2023.
[12] Mahiar Mahjoub, Loretta Sioson, Amy Sheen, Mahsa Ahadi, Angela Chou, Anthony J Gill, and Talia L Fuchs. Predicting survival in colorectal carcinoma after curative resection: a new prognostic nomogram. Pathology, 54(1) :79-86, 2022.
[13] Luana Greco, Federica Rubbino, Arianna Dal Buono, and Luigi Laghi. Microsatellite instability and immune response: from microenvironment features to therapeutic actionability-lessons from colorectal cancer. Genes, 14(6) :1169, 2023.
[14] Midhun Malla, Jonathan M Loree, Pashtoon Murtaza Kasi, and Aparna Raj Parikh. Using circulating tumor dna in colorectal cancer: current and evolving practices. Journal of Clinical Oncology, 40(24): 2846-2857, 2022.
[15] C Soueidy, A Zaanan, M Gelli, E Moati, C Gallois, V Taly, P Laurent-Puig, L Benhaim, and J Taieb. Clinical impact of circulating tumor dna to track minimal residual disease in colorectal cancer patients. hopes and limitations. ESMO Gastrointestinal Oncology, 4 :100068, 2024.
[16] Taane G Clark, Michael J Bradburn, Sharon B Love, and Douglas G Altman. Survival analysis part i: basic concepts and first analyses. British journal of cancer, 89(2) :232-238, 2003.
[17] David R Cox. Regression models and lifetables. Journal of the Royal Statistical Society: Series B (Methodological), 34(2): 187-202, 1972.
[18] Alexia lasonos, Deborah Schrag, Ganesh V Raj, and Katherine S Panageas. How to build and interpret a nomogram for cancer prognosis. Journal of clinical oncology, 26(8) :1364-1370, 2008.
[19] Tsuyoshi Konishi, Yoshifumi Shimada, Meier Hsu, Lauren Tufts, Rosa Jimenez-Rodriguez, Andrea Cercek, Rona Yaeger, Leonard Saltz, J Joshua Smith, Garrett M Nash, et al. Association of preoperative and postoperative serum carcinoembryonic antigen and colon cancer outcome. JAMA oncology, 4(3) :309-315, 2018.
[20] Wendy R Muñoz-Montaño, Horacio N López-Basave, Alison Castillo-Morales, Carolina Castillo-Morales, Karen Sánchez-Trejo, Rodrigo Catalán, Consuelo Diaz-Romero, Leonardo S Lino-Silva, Andrea Maliachi- Diaz, Erika Ruiz-Garcia, et al. Persistent high levels of carcinoembryonic antigen after tumor resection are associated with poorer survival outcomes in patients with resected colon cancer. BMC cancer, 23(1) :678, 2023.
[21] Samidha Borkar, Swarupa Chakole, Roshan Prasad, and Spandan Bansod. Revolutionizing oncology: A comprehensive review of digital health applications. Cureus, 16(4), 2024.
[22] Michael I Jordan and Tom M Mitchell. Machine learning: Trends, perspectives, and prospects. Science, 349(6245) :255-260, 2015.
[23] Bastian Pfeifer, Andreas Holzinger, and Michael G Schimek. Robust random forest-based all-relevant feature ranks for trustworthy ai. In Challenges of Trustable AI and Added-Value on Health, pages 137-138. IOS Press, 2022.
[24] Mahnaz Vahmiyan, Mohammadtaghi Kheirabadi, and Ebrahim Akbari. Feature selection methods in microarray gene expression data: A systematic mapping study. Neural Computing and Applications, 34 (22) :19675-19702, 2022.
[25] Abrar Yaqoob, Rabia Musheer Aziz, Navneet Kumar Verma, Praveen Lalwani, Akshara Makrariya, and Pavan Kumar. A review on nature-inspired algorithms for cancer disease prediction and classification. Mathematics, 11(5) :1081, 2023.
[26] de la FOUCHARDIÈRE, C., Les nouvelles recommandations sur l'usage des biomarqueurs dans la prise en charge des cancers colorectaux.
[27] https://ressources-aura.fr/wp-content/uploads/2021/12/Evaluation-du-statut-MMR-Tumoral-synthese-2021.pdf
[28] Taieb, J., Svrcek, M., Cohen, R., Basile, D., Tougeron, D. and Phelip, J.M., 2022. Deficient mismatch repair/microsatellite unstable colorectal cancer: Diagnosis, prognosis and treatment. European Journal of Cancer, 175, pp.136-157.
[29] https://has-sante.fr/upload/docs/application/pdf/2021-09/rapport_tests_compagnons.pdf
[30] Lv, Z., Liang, Y., Liu, H. and Mo, D., 2021. Association of chemotherapy with survival in stage II colon cancer patients who received radical surgery: a retrospective cohort study. BMC cancer, 21, pp.1-11.
[31] Cottin, A., Pecuchet, N., Zulian, M., Guilloux, A. and Katsahian, S., 2022. IDNetwork: A deep illness-death network based on multi-state event history process for disease prognostication. Statistics in Medicine, 41(9), pp.1573-1598.
[32] Cottin, A., Zulian, M., Pécuchet, N., Guilloux, A. and Katsahian, S., 2024. MS-CPFI: A model-agnostic Counterfactual Perturbation Feature Importance algorithm for interpreting black-box Multi-State models. Artificial Intelligence in Medicine, 147, p.102741.

### SUMMARY

It is therefore provided a computer-implemented method for computing a score representing a percentage of occurrence of a clinical event in a period of time. The method comprises obtaining a database. The database includes a list of P characteristics within a vector *X*, associated with the clinical event. Each characteristic (*p,* for *p* = {1,··· , *P*}) has a respective set of modalities (*pⱼ* for *j* = {1, ···, *nₚ*}*,* with *nₚ* the number of modalities of the characteristic *p*). The database includes, for each characteristic (*p*) of the list, a set of modality weights. Each modality weight (*α*(*pⱼ* )) is associated with a respective modality (*j*) of the characteristic (*p*) and quantifies its effect on the percentage of occurrence of the clinical event. The method comprises obtaining, for a given patient, the modalities corresponding to a subset of the characteristics in the list (*p* ∈ *X**, wherein *X** *⊂ X*)*.* The method comprises extracting, from the database, the modality weight associated with each modality obtained for the patient. The method comprises computing the score (s*coreₚₐₜᵢₑₙₜ*) for the given patient by summing the extracted modalities weights, using the formula *scoreₚₐₜᵢₑₙₜ* = Σ*ₚⱼ* ∈*_{X*} α*(*pⱼ*)*.* The method comprises computing a dispersion range associated with the computed score.

The method may comprise one or more of the following:
- The dispersion range comprises a maximum value (*Iₘₐₓ*) and a minimum value (*Iₘᵢₙ*). The list of characteristics (*p* ∈ *X*) includes at least one remaining characteristic (*p* ∉ *X**) excluded from the subset of characteristics (*X**) obtained for the patient. The computing of the dispersion range comprises:
   o computing (S43) the maximum value (*Iₘₐₓ*) by adding to the score the highest modality weight (*α*(*p⁺*)) of each remaining characteristic *p* ∉ *X*,* using the following formula: ${I}_{max} = {score}_{patient} + {\sum }_{p ∉ {X}^{∗}} α \left({p}^{+}\right) ;$ and/or
   o computing (S44) the minimum value (*Iₘᵢₙ*) by adding to the score the lowest modality weight (*α*(*p*⁻)) of each remaining characteristic *p* ∉ *X**, using the following formula: ${I}_{min} = {score}_{patient} + {\sum }_{p ∉ {X}^{∗}} α \left({p}^{-}\right) .$
- The method further comprises scaling (S50) the score and/or the dispersion range by applying a min-max scaler between a first value (e.g., 0) and a second value higher than the first value (e.g., 1).
- The method further comprises displaying (S60) a first graphical representation representing the score and the dispersion range on a scale ranging from the first value (0%) to the second value (100%).
- The scale on which the score is displayed is subdivided into a low risk area, an intermediate risk area, and a high risk area. Boundaries between the low risk area, the intermediate risk area and the high risk area correspond to 0.33 and 0.66 quantiles of retrospective data.
- The method further comprises displaying (S70) a second graphical representation indicating a relative contribution of each characteristic of the subset on the score computed for the patient.
- The method further comprises computing (S80) the relative contribution of each characteristic. The computing of the relative contribution of a given characteristic *p* comprises:
   o computing a contribution of the characteristic *p* using the following formula:
   ∘ 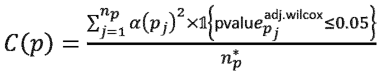
   o wherein:
      ▪ P is the total number of characteristics;
      ▪ *nₚ* is the number of modalities associated with the characteristic *p* (1 ≤ *p* ≤ *P*);
      ▪ ${n}_{p}^{∗}$ is the number of significant modalities associated to the characteristic p, with 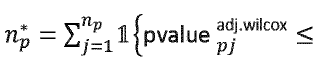 0.05} with 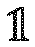 the indicator function;
      ▪ *α*(*pⱼ*) is the modality weight associated with the modality j of the characteristic p (1 ≤ p ≤ *P,* 1 ≤ j ≤ *nₚ*); and
   o computing the relative contribution of the characteristic p using the following formula: $RC \left(p\right) = \frac{C \left(p\right)}{{\sum }_{i = 1}^{P} C \left(i\right)} .$
- The second graphical representation further indicates an impact of each modality obtained for the patient on the computed score, for example by using a gradient color.
- The set of modality weights of each characteristic are determined by interpreting a model modeling a multi-state process of a disease progression comprising states and transitions between the states. The clinical event corresponds to a given transition of the multi-state process.
- The model is a Cox model. The modality weights correspond to the coefficients of the Cox model;
- The model is a deep learning neural network, each modality weight associated with a modality representing an average impact of the modality on transition probabilities computed by the model over a population of patients; and/or
- The method further comprises determining (S90) significant modalities of each characteristic by:
   ∘ obtaining a p-value for each modality weight, for example by applying a Wilcoxon test; and
   ∘ filtering the obtained p-values using a threshold.

It is further provided a computer program comprising instructions which, when the program is executed by a computer, cause the computer to carry out the method.

It is further provided a computer readable storage medium having recorded thereon the computer program.

It is further provided a system comprising a processor coupled to a memory. The memory has recorded thereon the computer program. The system may further comprise a graphical user interface.

It is further provided a device comprising a data storage medium having recorded thereon the computer program.

The device may form or serve as a non-transitory computer-readable medium, for example on a SaaS (Software as a service) or other server, or a cloud based platform, or the like. The device may alternatively comprise a processor coupled to the data storage medium. The processor may execute the instructions of the computer program stored on the data storage medium to cause the device to carry out the method and/or the method of use. The device may thus form a computer system in whole or in part (e.g. the device is a subsystem of the overall system). The system may further comprise a graphical user interface coupled to the processor.

### BRIEF DESCRIPTION OF THE DRAWINGS

Non-limiting examples will now be described in reference to the accompanying drawings, where:
- FIG. 1 shows an example of a nomogram.
- FIGs. 2 and 3 show flowcharts of examples of the method;
- FIG. 4 illustrates an example of multi-state process;
- FIG. 5 shows examples of characteristics;
- FIG. 6 shows examples of modality weights;
- FIGs. 7 to 9 illustrate examples of first graphical representations;
- FIG. 10 illustrates an example of relative contributions computed for characteristics;
- FIGs. 11 and 12 illustrate examples of second graphical representations; and
- FIG. 13 shows an example of the system.

### DETAILED DESCRIPTION

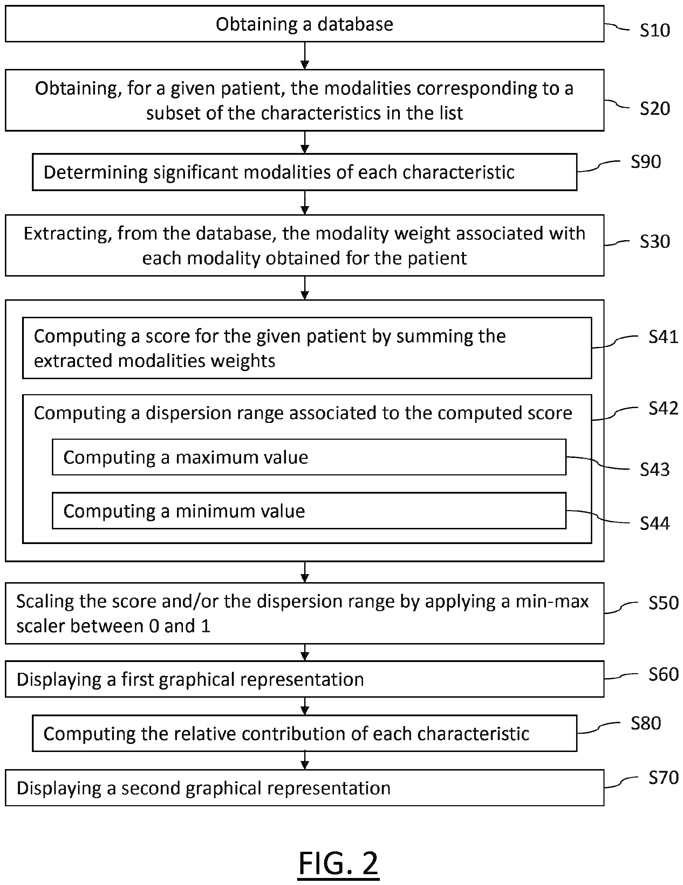

With reference to the flowchart of FIG. 2, it is provided a computer-implemented method for computing a score representing a percentage of occurrence of a clinical event in a period of time. The method comprises obtaining (S10) a database. The database includes a list of P characteristics within a vector X, associated with the clinical event. Each characteristic (*p*, for *p* = {1,··· *,P*}) has a respective set of modalities (*pⱼ* for *j* = {1,···,*nₚ*}, with *nₚ* the number of modalities of the characteristic *p*). The database includes, for each characteristic (*p*) of the list, a set of modality weights. Each modality weight (*α*(*pⱼ* )) is associated with a respective modality (j) of the characteristic (p) and quantifies its effect on the percentage of occurrence of the clinical event. The method comprises obtaining (S20), for a given patient, the modalities corresponding to a subset of the characteristics in the list (*p* ∈ *X*,* wherein X* ⊂ *X*)*.* The method comprises extracting (S30), from the database, the modality weight associated with each modality obtained for the patient. The method comprises computing (S41) the score (*scoreₚₐₜᵢₑₙₜ*) for the given patient by summing the extracted modalities weights, using the formula *scoreₚₐₜᵢₑₙₜ =* Σ*_{pⱼ ∈X*} α*(*pⱼ* )*.* The method comprises computing (S42) a dispersion range associated with the computed score.

Such a method forms an improved solution for computing a score representing a percentage of occurrence of a clinical event for a patient.

Notably, the method enables flexible score computation, as it allows for the calculation of a score even when only a subset of the characteristics in the list is available for a given patient. This ensures that the method remains applicable regardless of the completeness of patient data, making it suitable for real-world scenarios where some characteristics may be missing.

Moreover, the method allows for an evaluation of the relevance of the computed score by computing a dispersion range associated with the score. The dispersion range quantifies the variability of the score based on the available modalities, providing an indication of confidence in the result. A narrow dispersion range suggests that the computed score is reliable, whereas a wider range highlights greater uncertainty due to missing characteristics.

By combining these advantages, the method allows both adaptability and reliability. It allows computing a score with partial data in various clinical contexts while also assessing whether the computed score is sufficiently precise or if additional patient information is required for a more reliable evaluation. Based on the computed dispersion range, further investigation may be planned by the medical practitioner to improve the accuracy of the score. Ultimately, this approach optimizes efforts and resources in patient prognosis assessment.

Additionally, the computation of the score and the dispersion range is carried out automatically by a computer system. This ensures a deterministic and accurate assessment of the percentage of occurrence of a clinical event for a patient. The automation of the process enhances the ergonomics of the method, as a user, such as a medical doctor, only needs to provide the patient's modalities, and the system automatically computes and delivers the score along with its associated dispersion range. The method may be implemented as a platform for automatically computing a score and its associated dispersion range for a patient. By eliminating manual calculations, the method improves usability and efficiency in a clinical setting, allowing practitioners to focus on decision-making rather than computational tasks.

The method is computer-implemented. This means that steps (or substantially all the steps) of the method are executed by at least one computer, or any system alike. Thus, steps of the method are performed by the computer, possibly fully automatically, or, semi-automatically. In examples, the triggering of at least some of the steps of the method may be performed through user-computer interaction. The level of user-computer interaction required may depend on the level of automatism foreseen and put in balance with the need to implement user's wishes. In examples, this level may be user-defined and/or pre-defined.

A typical example of computer-implementation of a method is to perform the method with a system adapted for this purpose. The system may comprise a processor coupled to a memory the memory having recorded thereon a computer program comprising instructions for performing the method. The memory may also store the database. The memory is any hardware adapted for such storage, possibly comprising several physical distinct parts (e.g. one for the program, and possibly one for the database). The system may comprise a graphical user interface (GUI) for displaying to a user representations of data computed and/or provided and/or obtained in the course of the method (such as the computed score and dispersion range).

By "database", it is meant any collection of data (i.e. information) organized for search and retrieval (e.g. a relational database, e.g. based on a predetermined structured language, e.g. SQL). When stored on a memory, the database allows a rapid search and retrieval by a computer. Databases are indeed structured to facilitate storage, retrieval, modification, and deletion of data in conjunction with various data-processing operations. The database may consist of a file or set of files that can be broken down into records, each of which consists of one or more fields. Fields are the basic units of data storage. Users may retrieve data primarily through queries. Using keywords and sorting commands, users can rapidly search, rearrange, group, and select the field in many records to retrieve or create reports on particular aggregates of data according to the rules of the database management system being used.

In the case of the method, the database comprises a list of P characteristics associated with the clinical event. This list may be structured within a vector X, which may be stored in the database. Additionally, the database may include lists of characteristics associated with other clinical events. By clinical event, it is meant a state or transition that characterizes a patient's health status over time. This may, for example, refer to a state or transition within a multi-state model representing the progression of a patient's condition. For example, a clinical event may correspond to a transition between different health states, such as from a disease-free state to an illness state or from an illness state to death in an illness-death model.

The lists of characteristics may be identical or different depending on the clinical events to which they are associated. The characteristics associated with each clinical event may be predefined based on medical knowledge, statistical analyses, or expert-driven selection criteria. The database may support dynamic updates to include newly identified characteristics or refine existing ones based on evolving clinical research.

Each characteristic (p, for *p* = {1,··· , *P*}) has a respective set of modalities (*pⱼ* for *j* = {1, ··· , *nₚ*}, with *nₚ* the number of modalities of the characteristic *p*). This means that, for each patient, a characteristic may assume one of the modalities from its respective set. The set of characteristics may include more than three characteristics, and potentially more than ten or even twenty-five characteristics. These characteristics may be categorical, ordinal, or numerical, depending on their nature. Categorical characteristics, such as sex, blood type, or general health status, represent discrete values. Ordinal characteristics, such as severity level or disease stage, follow an inherent ranking. Numerical characteristics, such as age, height, weight, or blood pressure, may be expressed as predefined ranges or continuous values. The association between characteristics and their respective modalities may be stored in a structured format within the database, allowing efficient retrieval and processing.

Each characteristic may be medical, meaning it relates to the physiology of the patient. These characteristics may represent the general state of the patient, the condition of the patient with respect to an illness, or the overall progression of the disease, such as tumor size or cancer stage in oncology. For categorical and ordinal characteristics, the actual value assigned to a patient corresponds to one of a predefined set of potential values, whereas for numerical characteristics, the value is a real number that falls within a predefined interval of potential values.

Each modality of a characteristic may be associated with a modality weight. When the database includes modality weights for multiple clinical events, the weight assigned to a given modality may vary depending on the clinical event. This means that, for each clinical event among a plurality of clinical events, and for each characteristic in the list associated with that clinical event, the database stores a respective set of modality weights, with each weight corresponding to a specific modality of the characteristic.

For each given clinical event, a modality weight associated with a given modality quantifies the effect of that given modality on the percentage of occurrence of that given clinical event. It means that the modality weight represents the relative contribution of the given modality in influencing the likelihood of the given clinical event, with higher or lower absolute weights indicating stronger or weaker associations. For example, if the clinical event is the occurrence of a stroke, the characteristic "blood pressure level" may have modalities such as "normal", "elevated", and "hypertensive", each assigned a weight reflecting its impact on stroke risk. A higher weight for the "hypertensive" modality would indicate a stronger correlation with stroke occurrence compared to the "normal" modality. Similarly, in a multi-state disease progression model, a characteristic such as "tumor grade" may have modalities like "low," "intermediate," and "high", with increasing weights reflecting the greater likelihood of transitioning to an advanced disease stage. When the clinical event corresponds to a transition to another state in a disease, such as progression through different cancer stages, the modality weights may be positive if they promote the transition or negative if they inhibit it. The absolute value of a weight may be proportional to the magnitude of its influence on these opposing tendencies, meaning that stronger facilitating or inhibiting effects will be reflected in higher absolute values.

For each clinical event, the list of characteristics, as well as the modality weight associated with each modality of said characteristics, may be predetermined and established in any manner. The method may include the determining of the list of characteristics and modality weights of each clinical event, or they may be determined prior to the executing of the method.

In examples, the list and the modality weights may have been automatically determined through computational methods, such as by interpreting a model modeling the progression of a disease as a multi-state process. For example, the disease may be a cancer disease, for example a breast cancer, or a disease characterized by an intermediate progression state(s) and a final state. This multi-state process may comprise states, and transitions between these states. Each clinical event may in that case correspond to a given transition of this multi-state process.

In examples, the model modeling the progression of the disease may be a statistical model, such as the Cox model. The model may for example be the Cox proportional hazard - P.H. - model disclosed in the document [17]. In that case, the modality weights may be the coefficients of this Cox model. They are statistically determined.

Alternatively, the model may be a deep learning neural network, such as the multi-state model (referred to as the "IDNetwork" model in the following) described in the European patent application EP 4 057 297 A1 published on 14.09.2022, which is incorporated herein by reference. In particular, the multi-state model may be the multi-state model described from p.9, li.11 to p.10, li.8 of the description as filed of this European patent application EP 4 057 297 A1. In examples, the multi-state model may be an illness-death model. In that case, the multi-state model may comprise three states ("healthy", "relapsed" or "diseased" and "death") and three transitions between these states (i.e., a first transition between the state "healthy" and the state "relapsed" or "diseased", a second transition between state "relapsed" or "diseased" and the state "death" and a third transition between the state "healthy" and the state "death"). For example, the multi-state model may be the illness-death model described in the European patent application EP 4 057 297 A1 (referred to as the "IDNetwork" model in the following), which is incorporated herein by reference. In particular, the multi-state model may be the illness-death model described in p.10 from li.9 to 32 or from p.16, li.9 to p.18, li.11 of the description as filed of this European patent application EP 4 057 297 A1. In other examples, the multi-state model may comprise more than three states. In that case, the multi-state model may comprise a transition between each pair of states. Each transition is specific between two respective states (i.e., the two respective states of the pair).

The neural network may have the architecture of the function that is described from p.12, li.27 to p.14, li.29 of this European patent application EP 4 057 297 A1. The neural network may comprise a covariate-shared subnetwork and/or a transition-specific subnetwork per transition. The covariate-shared subnetwork may comprise a respective fully connected neural network. Alternatively or additionally, at least one transition-specific subnetwork may comprise a fully connected neural network. The covariate-shared subnetwork may comprise a respective non-linear activation function. Alternatively or additionally, at least one transition-specific subnetwork comprises a respective non-linear activation function. Each transition-specific subnetwork may be followed by a softmax layer. The multi-state model may comprise competing transitions, and the transition-specific subnetworks of the competing transitions may share a common softmax layer.

When the multi-state model is a deep learning neural network, the list of characteristics and the modality weights may for example be determined using the method disclosed in the European patent application EP 4 386 767 A1, published on June 19, 2024, which is incorporated herein by reference. The list of characteristics may be the one identified at step S41 in this method. The method disclosed in this application is hereinafter referred to as the MS-CPFI method.

In the MS-CPFI method, clinical events correspond to the transitions within the states of the multi-state model. For each transition (i.e., each clinical event), the model is trained to compute transition probabilities for patients during a follow-up period. The MS-CPFI method computes the modality weights by interpreting the transition probabilities computed by the model over a population of patients. For each characteristic, the modality weight of each modality may be computed analogously than the prediction-based feature importance computed for the baseline value (i.e., a modality) at step S30 in the European patent application EP 4 057 297 A1. The computational details may be found from p.31, li.25 to p.32, li.26 of the description as filed of this European patent application EP 4 057 297 A1.

Further details about the computing of the modality weights in that case are now provided. The model models a multi-state process conventionally associated with a set of transition intensities *hₖₗ* with (*k, l*) the transition from a state *k* to a state *l*. A cumulative counterpart *Aₖₗ* of this transition intensity, that is function of the time, represents a cumulative risk of transiting from state k to state l. *Âₖₗ*(*t*|*X*) is noted as the prediction obtained from the deep learning neural network.

The modality weights for a transition (k, l) may be computed based on the predictions of the function *Âₖₗ* by using the concept of feature importance and the notion of counterfactuals.

Each modality is considered as a counterfactual scenario. For a characteristic noted *p, x^{c}* is a counterfactual scenario that is for example if *x* is p: *x^{c} = pⱼ.*

The "feature importance" of a counterfactual scenario is defined as: ${FI}_{kl} \left(t, {x}^{c}\right) = {\overline{A}}_{kl} \left(t\left|{X}^{∖ x}, {x}^{c}\right.\right) - {\overline{A}}_{kl} \left(t\left|X\right.\right)$ wherein ${\overline{A}}_{kl} \left(t\right) = \frac{1}{n} {\sum }_{i = 1}^{n} {A}_{kl} \left(t\left|{X}_{i}\right.\right)$
is the reference cumulative transition intensity averaged over a population of n patients; and
for *X*\*x* the set of covariates excluding the feature x, ${\overline{A}}_{kl} \left(t\left|{X}^{∖ x}, {x}^{c}\right.\right) = \frac{1}{n} {\sum }_{i = 1}^{n} {A}_{kl} \left(t\left|{X}_{i}^{∖ x}, {x}^{c}\right.\right)$
is the cumulative transition intensity under a counterfactual scenario *x^{c}* (i.e. a feature modality).

Then, the weight of a counterfactual scenario *x^{c}* (i.e., of the modality *pⱼ*) may be computed for a transition (k, l) using the following formula: ${α}_{kl} \left({x}^{c}\right) = {\int }_{0}^{τ} {FI}_{kl} \left(t, {x}^{c}\right) dt$ wherein *τ* is the end of the period of time .

The database may further comprise the modalities of the characteristics for the considered population of n patients.

The obtaining S10 of the database may comprise constructing the said database. The construction of the database may comprise determining the list of characteristics and modality weights (e.g., as previously discussed) and recording the determined list and modality weights. Alternatively, the obtaining S10 of the database may comprise providing an access to an already constructed database. In that case, the database may have been constructed as previously discussed.

The step of obtaining S20 the modalities for a given patient is now discussed.

The given patient is a patient for whom the score is to be computed. The modalities obtained in step S20 are those that are available for this patient. These modalities may have been measured directly on the patient, for example, as a result of medical examinations, laboratory tests, or imaging studies conducted prior to the execution of the method. Such modalities may have been previously stored in memory, allowing retrieval at the time of execution. In this case, the obtaining S20 of the modalities may comprise retrieving these stored modalities from a database or an electronic health record. Alternatively, the modalities for the given patient may have been manually entered by a medical practitioner, such as a doctor, based on clinical assessments, observations, or historical patient data via an input interface of a computer system, and stored in a memory. This manual entry and storing may have taken place prior to the execution of the method, for example, during a medical consultation. In this case, the obtaining S20 of the modalities may comprise retrieving, for the memory, these practitioner-entered modalities received, prior to the method, through the input interface of the computer system. Additionally, the obtaining S20 of the modalities may involve a combination of automatic retrieval and manual input.

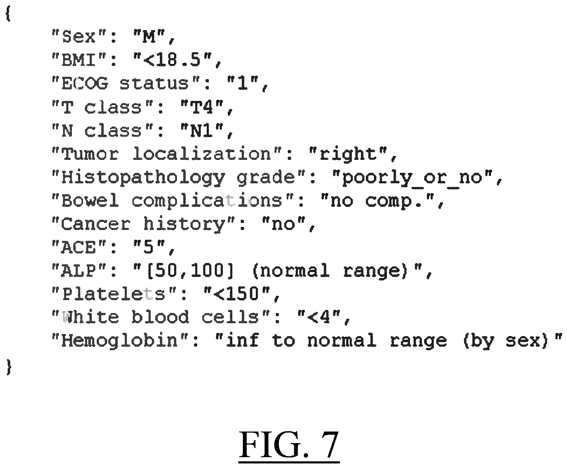

The obtained modalities correspond to a subset of the characteristics from the predefined list (*p* ∈ *X**, wherein *X** ⊂ *X*), meaning that for some characteristics, the patient's modality is known and may be used in the computation (see, e.g., the example of modalities obtained for the subset of characteristics illustrated in FIG. 7). For the remaining characteristics, the modality is unknown, for example, because the corresponding medical examination has not been performed, or the data is otherwise unavailable. Depending on the completeness of the patient's data, the obtained modalities may represent, for instance, at least half of the characteristics or even two-thirds of them, while the rest remain unknown.

The step of extracting S30 the modality weights may be performed in any manner. The extracting S30 of the modality weights may comprise querying the database using the set of obtained modalities as input and retrieving the corresponding weights. After the querying, the received weights may be recorded. When the method includes the step of determining S90 for significant modalities, the extracting step S30 may comprise extracting only the modality weights that correspond to those of the determined significant modalities (e.g., by including a filtering step). In that case, the score and dispersion range are computed by considering only significant modalities of the patient.

Then, the method comprises computing S41 the score for the given patient based on the extracted modality weights and computing S42 a dispersion range associated with this score. The score (denoted as *scoreₚₐₜᵢₑₙₜ*) is computed by summing the weights of the obtained modalities using the formula: ${score}_{patient} = {\sum }_{{p}_{j} ∈ {X}^{∗}} α \left({p}_{j}\right)$ where *X** represents the set of obtained modalities for the patient, and *α*(*pⱼ* ) denotes the modality weight associated with each obtained modality *pⱼ*. This summation quantifies the cumulative effect of the patient's known modalities on the occurrence probability of the clinical event.

In addition to the score, a dispersion range is computed to assess the variability and confidence level of the computed score. The dispersion range reflects the sensitivity of the score to the missing modalities and provides an indication of how much the result may vary if additional patient's data were available. A narrow dispersion range suggests that the computed score is stable and reliable, whereas a wider range indicates greater uncertainty due to missing information.

The score and the dispersion range may be computed sequentially, one after the other, or they may be computed simultaneously as part of the same processing step, depending on the implementation of the method.

The dispersion range may be computed in any manner. For example, it may be determined by calculating a maximum value (*Iₘₐₓ*) and a minimum value (*Iₘᵢₙ*), which define an interval reflecting the potential variation of the computed score due to missing modalities. The method accounts for the remaining characteristics (denoted as *p* ∉ *X*),* which are part of the list of characteristics *X* but were not obtained for the given patient. The computation estimates how the unknown modalities could influence the score if they were available.

The maximum value (*Iₘₐₓ*) is computed in step S43 by adding to the computed score (*scoreₚₐₜᵢₑₙₜ*) the highest modality weight *α*(*p⁺*) for each remaining characteristic (*p* ∉ *X**), using the following formula: ${I}_{max} = {score}_{patient} + {\sum }_{p ∉ {X}^{∗}} α \left({p}^{+}\right)$ where *α*(*p⁺*) represents the modality weight of the modality that most increases the risk of occurrence of the clinical event or risk of transition for each remaining characteristic. This means that *Iₘₐₓ* corresponds to the case where all missing characteristics take values that have the higher modality weight (i.e. the strongest negative influence of the risk of occurrence of the clinical event).

Similarly, the minimum value (*Iₘᵢₙ*) is computed in step S44 by adding to the computed score (*scoreₚₐₜᵢₑₙₜ*) the lowest modality weight *α*(*p*⁻) for each remaining characteristic (*p* ∉ *X*),* using the following formula: ${I}_{min} = {score}_{patient} + {\sum }_{p ∉ {X}^{∗}} α \left({p}^{-}\right)$ where *α*(*p⁻*) represents the modality weight of the modality that most decreases the risk of occurrence of the clinical event or risk of transition for each remaining characteristic. This means that *Iₘᵢₙ* corresponds to the case where all missing characteristics take values that have the lower modality weight (i.e. the strongest positive influence of the risk of occurrence of the clinical event).

The dispersion range [*Iₘᵢₙ*, *Iₘₐₓ*] quantifies the uncertainty introduced by missing data, providing an interval in which the actual score would lie if all patient data were available. The computation of *Iₘₐₓ* and *Iₘᵢₙ* may be performed separately or simultaneously, depending on the implementation. This may be done using database queries that retrieve the highest and lowest modality weights for each missing characteristic. The highest and lowest modality weights for each characteristic may for example be stored in a table in the database.

In examples, the method may further comprise a scaling step S50, in which the computed score and/or the dispersion range are transformed using a min-max scaling technique. This step ensures that the computed values are normalized within a predefined range, improving their interpretability and facilitating comparisons across different patients or clinical contexts.

In this step, the score and/or dispersion range are scaled between a first value (e.g., 0) and a second value higher than the first value (e.g., 1). The transformation may be performed using a min-max scaler, which maps each raw value to a specified range while preserving the relative differences between them.

The scaling may be applied to each value (y, which may be the *scoreₚₐₜᵢₑₙₜ, Iₘᵢₙ,* or *Iₘₐₓ*) using the following formula: ${y}_{scaled} = \frac{y - {y}_{min}}{{y}_{max} - {y}_{min}} \times \left({V}_{2}-{V}_{1}\right) + {V}_{1}$ wherein y represents the value being scaled (either the score or one of the dispersion range bounds), *yₘᵢₙ* and *yₘₐₓ* are predefined reference values, representing the minimum and maximum possible values for the score in the given dataset or clinical model, and *V*₁ and *V*₂ are the first and second values of the target range (e.g., 0 and 1).

A transformation in percentage may be applied to each scaled value if the target range is [0, 1] using the following formula : *y_{scaled}* × 100.

In examples, the method may further comprise a displaying step S60, in which a first graphical representation of the score and the dispersion range is displayed on a scale ranging from a first value (e.g., 0%, corresponding to no occurrence probability) to a second value (e.g., 100%, corresponding to full occurrence probability). The graphical representation may be displayed on a screen connected to the computing system performing the calculations. This screen may be integrated into a computer workstation, a tablet, or a medical device, allowing the results to be easily accessed in a clinical setting. The representation is typically displayed for a medical practitioner, such as a doctor, who can analyze the results and use them to support clinical decision-making.

The graphical representation may comprise a (e.g., horizontal) line or scale, visually representing the range from 0% to 100%. The graphical representation may comprise a point or marker on the scale, indicating the computed score for the given patient. The graphical representation may comprise a graphical interval (e.g., a shaded bar, a horizontal bracket, or a color gradient), representing the dispersion range. This interval visually communicates the possible variability of the score due to missing modalities, with narrower intervals suggesting more confidence in the computed score and wider intervals indicating greater uncertainty.

By displaying this graphical representation, the medical practitioner (e.g., the doctor) may immediately assess the computed score in the context of its variability and make an informed decision accordingly. For example, if the score falls within a high-probability range, the practitioner may consider proceeding with a specific medical intervention. Conversely, if the dispersion range is too wide, indicating uncertainty due to missing data, the practitioner may decide to request additional tests or gather more patient information before making a clinical decision.

In examples, the scale on which the score is displayed is subdivided into three risk areas: a low-risk area, an intermediate-risk area, and a high-risk area. The boundaries between these areas are determined using the 0.33 and 0.66 quantiles of retrospective patient data, ensuring that the classification reflects past observations. The low-risk area corresponds to scores below the 0.33 quantile, the intermediate-risk area includes scores between the 0.33 and 0.66 quantiles, and the high-risk area includes scores above the 0.66 quantile. This segmentation provides a more intuitive interpretation of the score, helping the medical practitioner quickly assess the patient's risk level. In particular, it allows the stratification into patient subgroups (low, intermediate or high risk).

In that case, the scale of the graphical representation may be divided into these three risk areas (e.g. using three different colors, such as green, orange and red), with the patient's score displayed as a marker on the scale, together with graphical element representing the dispersion range. This allows the practitioner to assess not only the estimated risk level but also the level of confidence in the result. If the entire dispersion range falls within the low-risk or intermediate-risk area, the practitioner may rule out a high-risk classification without further investigation. Alternatively, if the dispersion range overlaps with the high-risk area, there is a potential risk, even if the computed score itself is not in that area.

This visualization improves clinical decision-making by directly indicating whether additional tests or patient data collection are necessary. If the dispersion range is too wide, it suggests that the available patient data is insufficient, prompting the practitioner to seek further information before making a clinical decision. If the interval remains within a single risk category, the practitioner can make a more confident assessment, potentially avoiding unnecessary tests. Moreover, this representation can assist in adjusting the frequency of medical examinations based on the patient's risk level. For instance, a high-risk classification may warrant more frequent monitoring, while a low-risk classification could justify less frequent follow-ups, optimizing medical resources and patient management.

In examples, the method may further comprise displaying S70 a second graphical representation indicating the relative contribution of each characteristic of the subset to the score computed for the patient. This second graphical representation may be displayed as an alternative to and/or in addition to the first graphical representation (e.g., before or after the latter). The method may allow alternative or sequential display of the graphical representations.

For example, each graphical representation may be alternatively displayed upon user interaction, such as selecting an option, triggering a command, or navigating between different views. Additionally, the second graphical representation may be displayed automatically based on predefined conditions, such as when the computed score exceeds a certain threshold or when the dispersion range is wide, indicating a higher level of uncertainty.

The second graphical representation may be implemented using visual elements such as bar charts, pie charts, or color-coded indicators to illustrate the relative impact of each characteristic on the score. This visualization helps practitioners to better understand the key characteristics and modalities driving the patient's risk assessment. For example, the second graphical representation may be a pie chart, wherein each slice represents a characteristic from the subset used in the score computation. The size of each slice may be proportional to the relative contribution of the characteristic, meaning that characteristics with a greater influence on the score will have larger sections in the chart. This visual representation allows the medical practitioner to quickly identify the most influential characteristics in the computed score.

Additionally, each slice of the pie chart may be color-coded based on the impact of the obtained modality on the score. A gradient color scheme may be applied, where one color (e.g., blue or green) represents positive contributions (modalities increasing the score) and another color (e.g., red or orange) represents negative contributions (modalities decreasing the score). This enables the medical practitioner to not only see which characteristics are most influential but also whether their effect is increasing or decreasing the computed score, further improving risk assessment and clinical decision-making.

In examples, the method may further comprise, prior to the displaying of the second graphical representation, the computing S80 of the relative contribution of each characteristic. The method may initially comprise the computing of a (i.e., non-relative) contribution of each characteristic. The non-relative contribution of a given characteristic p may be computed using the following formula: 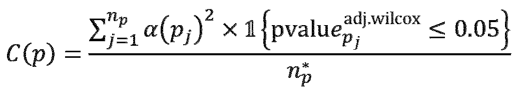 wherein:
- P is the total number of characteristics;
- *nₚ* is the number of modalities associated with the characteristic *p* (1 ≤ *p* ≤ *P*);
- ${n}_{p}^{∗}$ is the number of significant modalities associated to the characteristic p, with 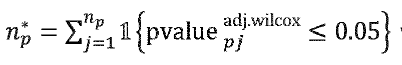 with 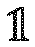 the indicator function;
- *α*(*pⱼ* ) is the modality weight associated with the modality *j* of the characteristic *p* (1 ≤ *p* ≤ *P,* 1 ≤ *j* ≤ *nₚ*)*.*

Then, the relative contribution of each characteristic may be computed based on the contributions computed for all the characteristics. The relative contribution of a characteristic *p* may be computed using the following formula: $RC \left(p\right) = \frac{C \left(p\right)}{{\sum }_{i = 1}^{P} C \left(i\right)}$ wherein:
- *C*(*i*) is the contribution of the characteristic *i;* and
- *RC*(*p*) is the relative contribution of the characteristic *p*.

In examples, the second graphical representation may solely represent the contribution of significant modalities of the given patient. The significant modalities of each characteristic may in that case be evaluated by the method, prior to the displaying of the second graphical representation, at step S90. In examples, the score and dispersion range may also be computed by considering only significant modalities of the patient. For example, the step of determining S90 for significant modalities may be performed prior to the extracting step S30, and in that case, the method may comprise extracting S30 only the modality weights that correspond to those of the determined significant modalities (e.g., by including a filtering step).

The determining S90 of the significant modalities of each characteristic may comprise the following steps. These steps are explained in the example of the MS-CPFI method. A first step thus comprises obtaining a p-value for each modality weight *pⱼ,* for example by applying a Wilcoxon test (e.g., as disclosed in https://fr.wikipedia.org/wiki/Test_de_Wilcoxon-Mann-Whitney). Indeed, several repetitions (e.g., 20) of the modality weight computation are performed using cross-validation. In other examples, another test may be used. For example, the Student test may be used when more repetitions are performed (e.g., 30).

Then, for each modality of each characteristic, the modality weights are estimated using a Monte Carlo median-based estimator, where the final estimate is obtained by taking the median of the modality weights across the several repetitions. To assess the statistical significance of this median estimate, a Wilcoxon test is applied, which statistically validates whether the estimate is significantly different from zero (i.e., whether the estimated alpha weight value is different from zero, meaning that the modality has a significant effect on the event risk). This test is conducted with a confidence level of 1 - 0.05 (typically 95% confidence).

Since a Wilcoxon test is performed for each modality of each variable, these tests may be not independent, introducing a statistical bias. In statistical analysis, this bias may be typically corrected by adjusting the p-values obtained from the Wilcoxon tests using an appropriate multiple testing correction procedure (see, e.g., the procedure disclosed in https://pmc.ncbi.nlm.nih.gov/articles/PMC6099145/). A second step may thus comprise adjusting the p-values obtained for each modality weight. In this case, the Benjamini-Hochberg procedure is applied to control the false discovery rate. The adjusted p-values are then used to determine whether a modality is statistically significant or not. In particular, the adjusted p-values (pvalue ${pvalue}_{pj}^{adj . wilcox}$) may be filtered, during a third step, using a threshold (e.g., 0.05).

In the example of the Cox model, significance of the p-values associated with the model coefficients may also be automatically calculated using a Wald test, which allows for determining the significance of the different modalities of each characteristic. This computation may be performed automatically in the output of a Cox model, as described in section 8.5 "Local tests" on page 263 of the following document: https://sistemas.fciencias.unam.mx/^{∼}ediaz/Cursos/Estadistica3/Libros/ 0a9X.pdf. In that case, p-values may be used directly (without adjustment). Indeed, the p-values are calculated from the same model and jointly. Thus, if a threshold of 0.05 is selected, all the tests performed in the model give a confidence level of 95%. The method may therefore filter the p-values directly using the threshold.

In examples, the method may be included in a treatment process of the patient which may comprise, after the executing of the method, determining a treatment plan, adapting an existing treatment, scheduling a follow-up visit, and/or conducting a surveillance phase with regular blood tests based on the computed score and dispersion range. For example, when the dispersion range exceeds a predefined threshold, this may indicate that the available patient data is insufficient, thereby prompting the scheduling of additional examinations, for example, to identify patient modalities that do not belong to the subset considered for score calculation, prior to making a clinical decision. Alternatively, for example when the dispersion range remains confined within a single risk category, the practitioner may perform a more reliable assessment, potentially avoiding unnecessary examinations. In such a case, the treatment process may comprise adjusting the frequency of medical examinations or therapeutic interventions (e.g., chemotherapy in the context of oncological treatment) based on the patient's assessed risk level.

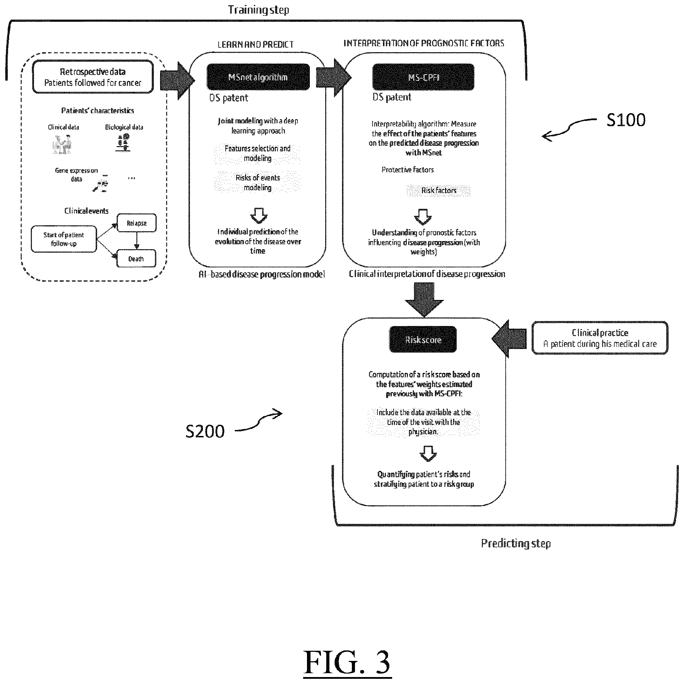

With reference to FIGs. 3 to 13, an example of implementation of the method is now presented.

In a context of medical decision support, it is proposed a pipeline comprising a method for computing a score representing the percentage of occurrence of a clinical event (e.g. relapse or death) in a period of time, for the stratification of patients into risk groups, by using two existing algorithms and one new-defined computation method of a risk score.

This pipeline is illustrated in FIG. 3 and is divided into two steps:
In a first training step S100, two existing algorithms are used:
- IDNetwork which uses retrospective survival (or multi-state) data and patients characteristics data in order to jointly model risks of clinical events related to a specific disease and disease characteristics;
- MS-CPFI which identify the prognostic effect (on average) of each disease characteristic in relation to each of the modelized clinical events. At the end of this step, the weights are defined, and the significant or non-significant modalities are determined.

In a second predicting step S200, the method is executed and computes a risk score for a specific patient (i.e., a patient that is not part of the retrospective data and that has a set of individual characteristics matching with at least one disease characteristics seen in the retrospective data (i.e., learned during the training step). This step allows also the stratification of the patient into a risk, as well as the interpretation of the patient's characteristics involved in the estimated score.

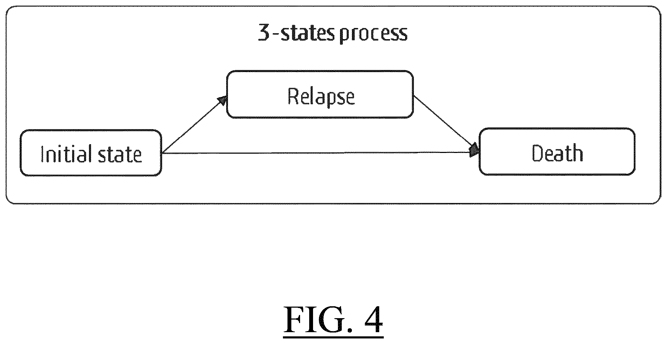

In this example, the disease is a cancer. The method considers a generalized 3-states relapse process model that models, from an initial state (state 0), the occurrence of a relapse (state 1) or a death (state 2) of cancer patients, also known as a multi-state or survival model. The process is irreversible and characterized by three transitions 0→1, 0→2, 1→2. The process is illustrated in FIG. 4.

In this example, if the problem is "What is the risk of having a cancer relapse (also called cancer recurrence)?", then the transition from the initial state to the state relapse (i.e. transition 0→1) will be studied in order to quantify the percentage of risk associated with this clinical event.

The algorithm IDNetwork is trained on this 3-states model (on retrospective patients' data comprising patients' characteristics - i.e. features with modalities - and survival data for a specific disease) and is interpreted with the MS-CPFI method (i.e. estimated modality weights on the specific clinical event).

The retrospective patient data used for determining the boundaries between the three risk areas (the low-risk area, the intermediate-risk area, and the high-risk area) may correspond to the dataset on which the modality weights (such as the MS-CPFI weights or the Cox model weights) are computed (used for the training in step S100). The definition of risk zones based on quantiles may be provided along with the set of weights during the training stage. The quantiles may be determined as follows. The quantiles may be determined using the training dataset, i.e., the data on which the MS-CPFI weights or Cox model weights are computed. The scores may be computed for all patients and by scaling them between 0 and 1. Based on these scores from the training phase, the 0.33 and 0.66 quantiles may be calculated, and their values may be stored in the database. The values of these quantiles (i.e., the boundaries of the different risk groups), may be obtained during step S10, similarly to the modality weights. The retrospective patient data used for the training in step S100 may consist of data with a clearly defined and specified disease description (e.g., stage III colon cancer with explicitly defined inclusion or exclusion criteria), ensuring that when computing a patient's score, the disease description falls within the spectrum defined in S100.

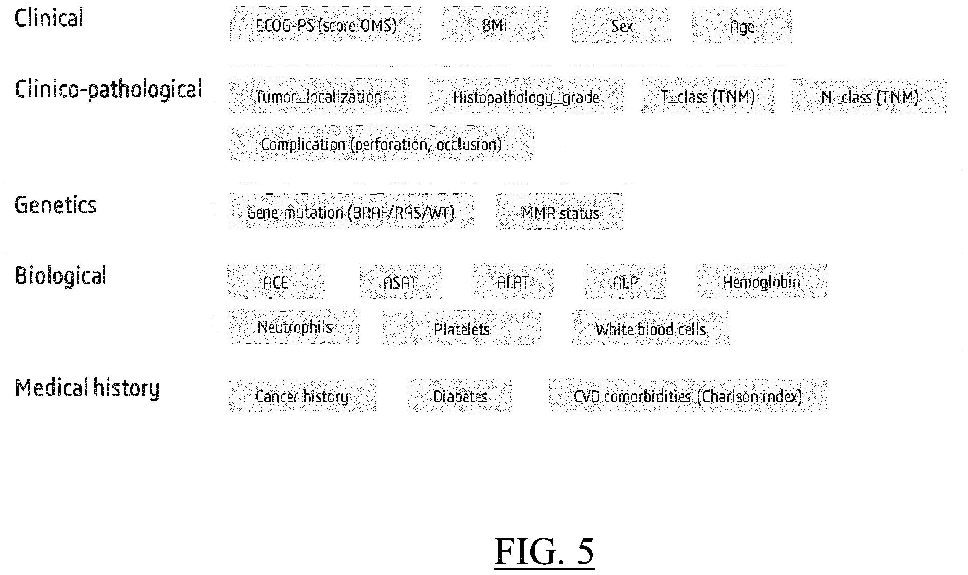

The method comprises obtaining a list of characteristics denoted by X and associated with clinical event prediction (in this example the cancer recurrence). Each characteristic (or feature, both terms being used interchangeably) comprises a set of modalities (i.e. a set of possible values); e.g. a feature "Sex" have the modalities "men" and "women", a feature "T_class" have the modalities "T1", "T2", "T3", "T4". This list of features and modalities being the ones used in the training step and comprising the most exhaustive disease characteristics; e.g. in this example, clinical, clinic-pathological, medical histories, biological, molecular and transcriptomic characteristics are obtained (the characteristics are illustrated in FIG. 5).

The method further comprises obtaining a set of characteristic weights obtained from the training step, each weight being associated to a characteristic (those weights are not used in the computing of the score. They are used after, to evaluate the contribution of each characteristic in the score).

The method further comprises obtaining a set of modality weights. The modality weights are obtained from the training step. Each weight is associated with a modality of feature. In this example, let us note *α*₀₁(*x^{pⱼ}* ) the weight of the modality j of the feature p for the transition 0→1.

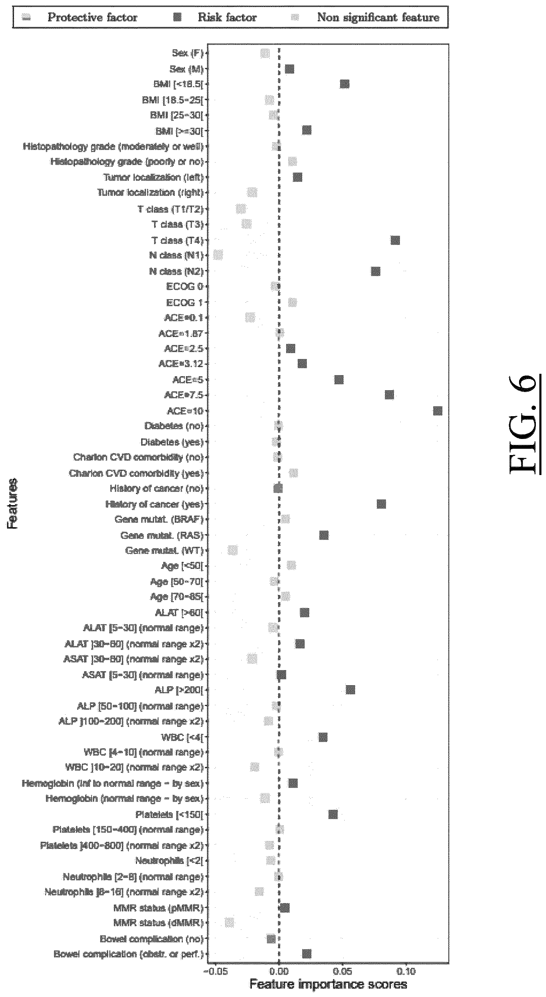

FIG. 6 illustrates an example of modality weights for an example list of characteristics. In particular, the figure presents a graphic showing the modality weight ("feature importance scores") associated with each modality of all the characteristics ("features") in the list. For example, the graphic includes the sex characteristic with the modality 'F' and the modality 'M'. For this clinical event, the figure indicates that the modality 'F' is identified as a risk factor, while the modality 'M' is considered as a protective factor, indicating that females have a higher risk of experiencing this clinical event compared to males. Modalities that are protective factors are illustrated in green, whereas modalities that are risk factors are illustrated in red. The figure also represents modalities determined by the method as non-significant in grey. For example, none of the modalities for the age or diabetes characteristics are significant.

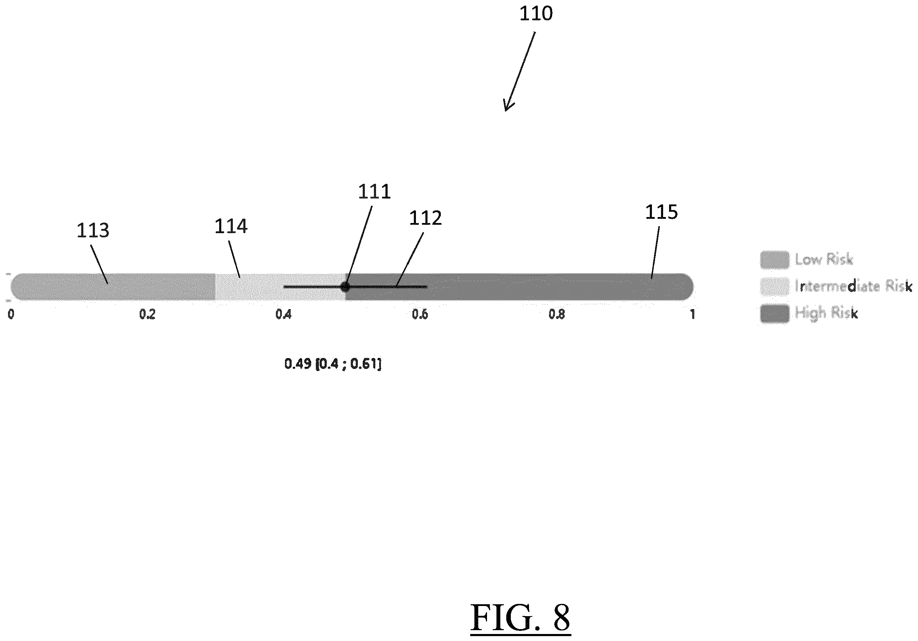

FIGs. 7 and 8 illustrate a first example of the displayed first graphical representation. In particular, FIG. 7 shows the modalities of the patient that are considered in this first example. For this patient, for at least one characteristic, the method comprises evaluating the modality associated with this at least one characteristic; e.g. in this example a set of the following modalities *X** ∈ *X* (where some of the features evaluated in the training step are not available).

For this patient, and the clinical event considered, the method computes, based on the modalities obtained for the patient:
- A score for the patient, the score being a sum of the modalities weights corresponding to the evaluated modalities:
   The formula is: ${score}_{patient}^{01} = {\sum }_{{p}_{j} ∈ {X}^{∗}} {α}_{01} \left({x}^{{p}_{j}}\right)$. The score is then scaled between 0 and 1 by learning the scaler on the retrospective data.
- A dispersion range associated with this score:
   If all the features in X are not available for the patient, then the risk score will be biased. In that case, the method proposes computing a dispersion range associated with this score. To do that, the idea is to create combinations of the missing features, i.e. to create several counterfactual-based versions of the patient by successively attributing the missing features by their counterfactuals ones (the ones defined in the MS-CPFI method) and then to compute the risk scores for each of these counterfactual-based versions of the patient. One alternative consists in computing all the combinations (i.e., the counterfactual-based versions of a patient), and determining the bounds for the dispersion range as follows: the lower bound of the dispersion range is the minimal risk score obtained on the counterfactual-based versions of the patient; and the upper bound of the dispersion range is the maximal risk score obtained on the counterfactual-based versions of the patient.

For the implementation, it is not required to compute all the combinations, as it is equivalent computing the bounds by considering the lowest and highest modality weights of the non-evaluated features only. Another alternative (more efficient) thus consists of computing the bounds of the dispersion range as follows:
- The minimum of the dispersion range (*Iₘᵢₙ*) may be computed by adding the lowest modality weight of the non-evaluated features to the score.
- The maximum of the dispersion range (*Iₘₐₓ*) being computed by adding the highest modality weight of the non-evaluated features to the score.

This dispersion range is therefore a deviation interval based on the min/max score the patient could have if all its features have been available.

As an illustration, let us take a simplest example than this example and consider a list of features X comprising "Sex" with the modalities "men" and "women", "T_class" with the modalities "T1", "T2", "T3", "T4", "N_class" with the modalities "N1", N2" and "cancer history" with the modalities "yes", "no". Each feature modality is associated with a weight noted *α*₀₁(feature_{modality} ) ; e.g. *α*₀₁ (Sexₘₑₙ ) for the modality "men" of the feature "Sex".

Let us consider a patient noted "patient*" following this simplest example with the features following modalities *X** ∈ *X* : {"Sex": "men", "T_class": "T4"}. The score of the patients is then computed as: ${score}_{{patient}^{∗}}^{01} = {α}_{01} \left({Sex}_{men}\right) + {α}_{01} \left({Tclass}_{T 4}\right) .$

The minimum (resp. maximum) of the dispersion range is then computed by adding the lowest (resp. the highest) modality weights of the non-evaluated features (i.e. "N_class" and "cancer history") to the score.

In this example, let consider for "N_class" the lowest weight modality (resp. the highest) is "N1" (resp. "N2"); for "cancer history" the lowest weight modality (resp. the highest) is "no" (resp. "yes"). The minimum of the dispersion range (noted *Iₘᵢₙ*) is computed as: ${I}_{min} = {score}_{{patient}^{∗}}^{01} + {α}_{01} \left({Nclass}_{N 1}\right) + {α}_{01} \left({CancerHistory}_{NO}\right)$

The maximum of the dispersion range (noted *Iₘₐₓ*) is computed as: ${I}_{max} = {score}_{{patient}^{∗}}^{01} + {α}_{01} \left({Nclass}_{N 2}\right) + {α}_{01} \left({CancerHistory}_{YES}\right)$

This illustration may be generalized. Let us introduce the following notations:
- *α*₀₁(*p⁺*) the highest modality weight of the feature p (with p included in the non-evaluated feautres of the specific patient (i.e. p *∉ X**).
- *α*₀₁(*p⁻*) the lowest modality weight of the feature p (with p included in the non-evaluated feautres of the specific patient (i.e. p ∉ *X**).

Then, for a patient noted "patient*", if the definition of the dispersion range is generalized with these notations, the minimum of the dispersion range may be computed using the following formula: ${I}_{min} = {score}_{{patient}^{∗}}^{01} + {\sum }_{p ∉ {X}^{∗}} {α}_{01} \left({p}^{-}\right)$

The maximum of the dispersion range may be computed as: ${I}_{max} = {score}_{{patient}^{∗}}^{01} + {\sum }_{p ∉ {X}^{∗}} {α}_{01} \left({p}^{+}\right)$

Then, the method may comprise associating a risk group to this score. Risk groups may be created based on the quantiles 0.33 and 0.66 (noted q_0.33 and q_0.66). These quantiles may be computed on the retrospective data:
- if the score of the patient is in the interval [0; q_0.33], then the group is "low risk";
- if the score of the patient is in the interval ]q_0.33 ; q_0.66], then the group is "intermediate risk";
- if the score of the patient is in the interval ]q_0.66 ; 1], then the group is "high risk".

FIG. 8 illustrates the first graphical representation 110 displayed to the user.

This first graphical representation 110 of the computed score and its dispersion range is displayed on a scale ranging from 0% (no occurrence probability) to 100% (full occurrence probability). This representation, which may appear on a screen connected to a computing system (such as a medical device) allows medical practitioners to quickly access and interpret results for the patient.

The first graphical representation 110 includes a horizontal scale with a marker 111 indicating the computed score and a graphical interval 112 illustrating the dispersion range. This interval visually conveys the variability of the score due to missing modalities, with narrower ranges indicating higher confidence and wider ranges reflecting greater uncertainty. The scale is divided into three risk areas: the low risk area 113, the intermediate risk area 114, and the high risk area 115, that are determined using the 0.33 and 0.66 quantiles of retrospective patient data. These areas are color-coded (e.g., green, orange, and red).

In this example, the marker 111 is between the intermediate risk area 114 and the high risk area 115, and the graphical interval 112 is above these two areas. In particular, the score of the patient is 49% with a dispersion range [40; 61] and the patient cannot be stratified in a unique risk group (i.e., he is between the "intermediate risk" and the "high risk" group). It can therefore not be deduced whether the patient belongs to the high risk area.

More patient' information are thus required for stratifying a patient in the right risk group. This incertitude can be unveiled with the addition of genetic biomarkers: in this example, this means that the physican could ask for genetic mutation research and MMR status research. In this example, this patient has the nRAS mutation and a proficient MMR (pMMR) status (121).

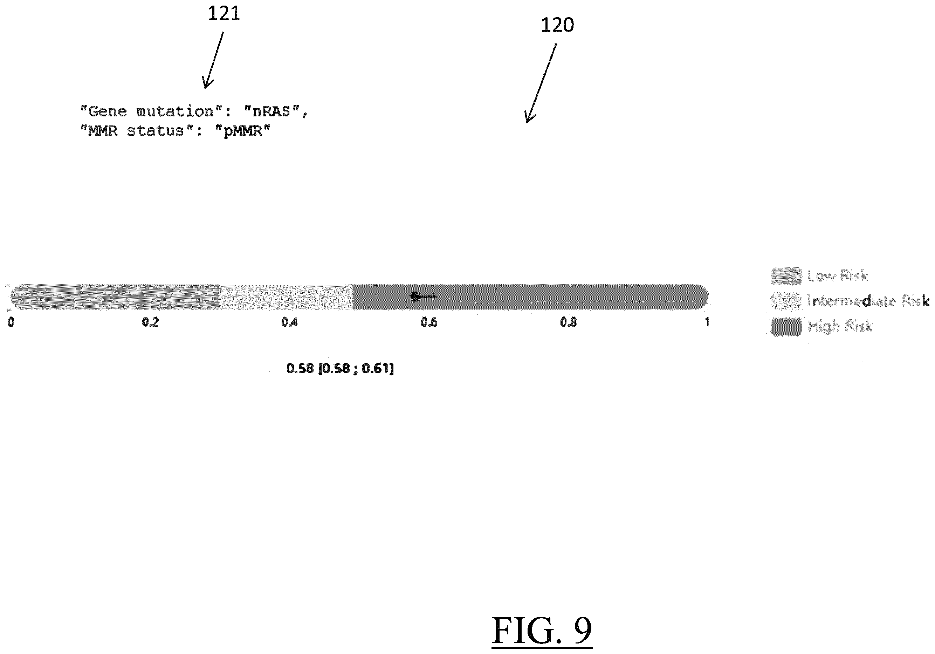

Then, in this example, according to this information, the risk score can be updated. The method is repeated with the additional information. The method updates the first graphical representation, and the new graphical representation 120 is illustrated in FIG. 9. Now, the score of the patient is 58% with a dispersion range [58; 61] and the patient can thus be stratified in the group "high risk". Moreover, the dispersion range is completely included in the high risk area, which signifies that further investigation would not change the patient category determined. It is therefore not relevant to search for additional features.

For allowing the clinician understanding the construction of the score (i.e. interpreting the features and the modalities of the patient that led to this score and to the assignment to this risk group), the method computes and displays a second graphical representation by using colors and based on the weights *α*₀₁(.) of the patient's modalities:
- A positive weight means that the modality is a risk factor (i.e. increase the risk of occurrence of the event). Therefore for positive weights, the method uses a gradient color of red. This gradient scale is constructed based on all the weights obtained from the MS-CPFI algorithm:
   ∘ The positive weights are mapped into a gradient color scale from light red to dark red.
   ∘ The highest positive weight is assigned dark red on the gradient color scale.
   ∘ The lowest positive weight is assigned light red on the gradient color scale.
   ∘ The other positive weights are assigned to a red shade that is proportional to their effect.
- A negative weight means that the modality is a protective factor (i.e. decrease the risk of occurrence of the event). Therefore for negative weights, a gradient color of green is used. This gradient scale is constructed based on all the weights obtained from the MS-CPFI algorithm:
   ∘ The negative weights (in absolute value) are mapped into a gradient color scale from light green to dark green.
   ∘ The highest negative weight (in absolute value) is assigned dark green on the gradient color scale.
   ∘ The lowest negative weight (in absolute value) is assigned light green on the gradient color scale.
   o The other negative weights are assigned a green shade that is proportional to their absolute effect.
- For a non-significant modality: a color grey is assigned on the feature (the statistical significance of the modality is obtained previously in the estimation with MS-CPFI with a Wilcoxon test for example and the use of adjusted p-values : then a modality j of a feature p is significant if its adjusted p-value, noted pvalue $\begin{matrix}adj . wilcox \\ pj\end{matrix}$, is less or equal than a threshold - usually 0.05).
- If all the modalities of a feature are non-statistically significant, then this feature will not be taken into account.

A sunburst plot that is composed of two nested pie plots is drawn following the two-color scales:
- A pie chart with the type of data.
- A pie chart with modality of the patient for each feature and the corresponding shade of red or green. If a feature is missing, then there is a blank color. In this pie chart, the width of the chart parts is proportional to the relative contribution of the feature to the model. This relative contribution is computed by considering the average effect of the feature across all its modalities, regardless of their sign). The method defines a relative importance score for each feature as follows.

First, the following notations are defined:
▪ P the number of features;
▪ *nₚ* the number of modalities associated to the feature p (1 ≤ *p* ≤ *P*);
▪ ${n}_{p}^{∗}$ the number of significant modalities associated to the feature p (1 ≤ *p* ≤ *P*), i.e. 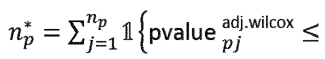 0.05} with 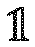 the indicator function ;
• *α*₀₁(*x^{pⱼ}*) the MS-CPFI score of the modality j of the feature p (1 ≤ *p* ≤ *P, 1* ≤ *j* ≤ *nₚ*)*.*

The relative contribution of the feature p is given by:

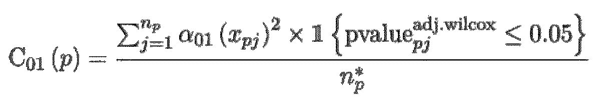

This is a quadratic meaning like formula.

The relative contribution of the feature p is then given by: ${RC}_{01} \left(p\right) = \frac{{C}_{01} \left(p\right)}{{\sum }_{p = 1}^{P} {C}_{01} \left(p\right)}$

For a feature with no significant features, the relative contribution is equal to 0 and this feature will not appear on the pie chart.

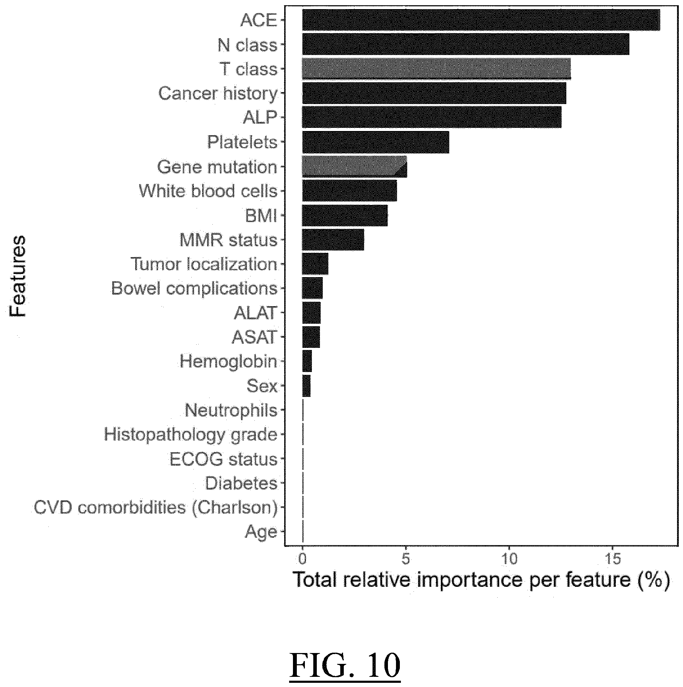

The relative contributions computed in this example are illustrated in FIG. 10.

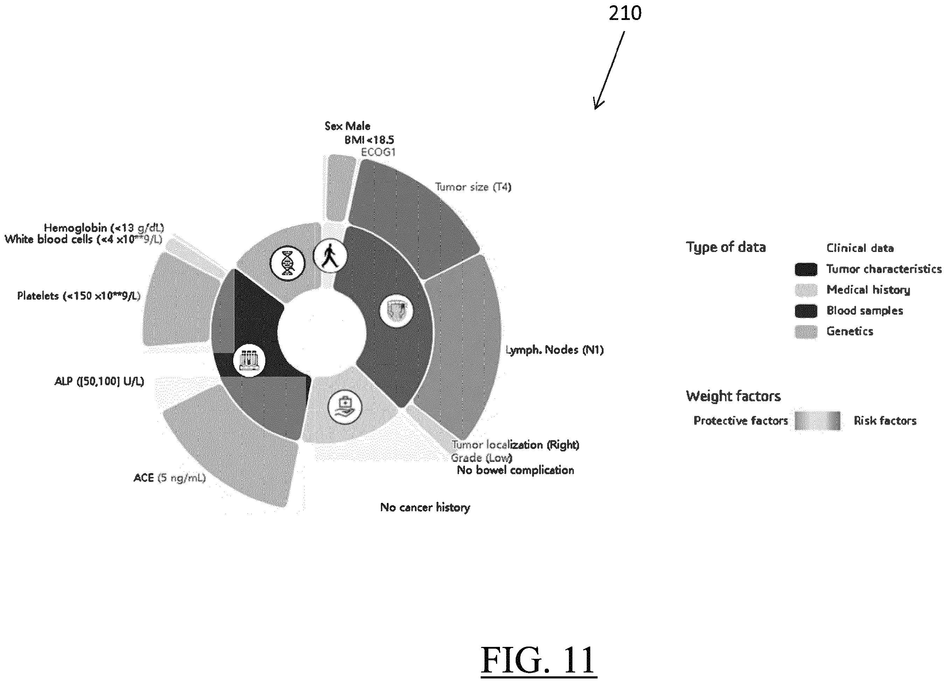

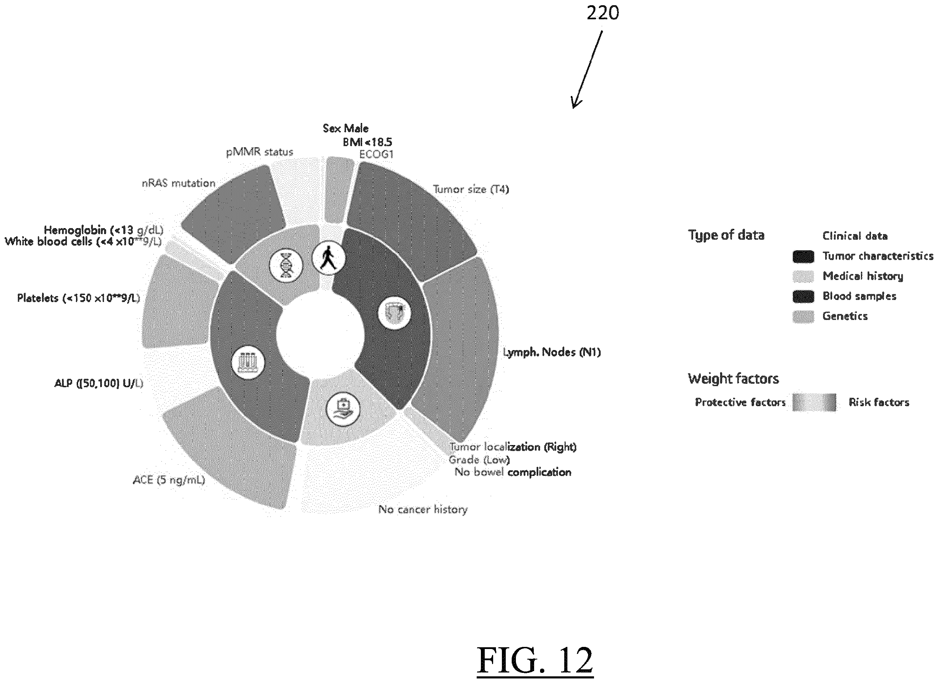

FIGs. 11 and 12 illustrate examples of second graphical representations. Especially, FIG. 11 illustrates the second graphical representation 210 with the modalities of the patient (given in FIG. 7) considered in the example of FIG. 8, and FIG. 12 illustrates the second graphical representation 220 updated with the modalities of the patient considered in the example of FIG. 9 (i.e., with the additional patient data given in 121).

The following information may be immediately deduced from these second graphical represents:
- The patient's N-class is N1: it is a high protective factor. The N-class is the feature with the highest contribution (i.e. with largest part is the pie).
- The patient is T4 (T-class): this modality is a high risk factor.
- The patient has a CEA level of 5 ng/mL, platelets <150x10e-9/L and is a male: the modalities are moderate risk factors.
- The nRAS mutation is a high risk factor and pMMR status is a moderate risk factor. Updating the patient's score by adding these features increases the risk of the patient and allows to stratify him in the "high risk" group.
- The method allows accurately visualizing that the patient cumulate several high and moderate risk factors that explain its assignment in the "high risk" group.

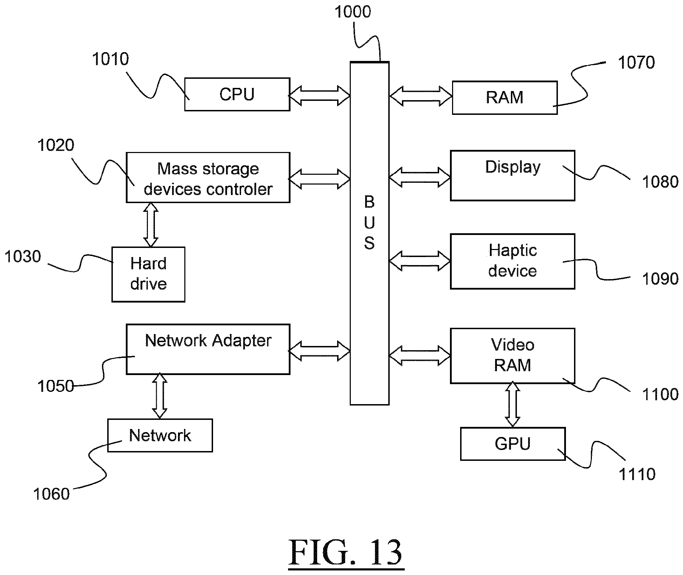

FIG. 13 shows an example of the system, wherein the system is a client computer system, *e.g*. a workstation of a user.

The client computer of the example comprises a central processing unit (CPU) 1010 connected to an internal communication BUS 1000, a random access memory (RAM) 1070 also connected to the BUS. The client computer is further provided with a graphical processing unit (GPU) 1110 which is associated with a video random access memory 1100 connected to the BUS. Video RAM 1100 is also known in the art as frame buffer. A mass storage device controller 1020 manages accesses to a mass memory device, such as hard drive 1030. Mass memory devices suitable for tangibly embodying computer program instructions and data include all forms of nonvolatile memory, including by way of example semiconductor memory devices, such as EPROM, EEPROM, and flash memory devices; magnetic disks such as internal hard disks and removable disks; magneto-optical disks. Any of the foregoing may be supplemented by, or incorporated in, specially designed ASICs (application-specific integrated circuits). A network adapter 1050 manages accesses to a network 1060. The client computer may also include a haptic device 1090 such as cursor control device, a keyboard or the like. A cursor control device is used in the client computer to permit the user to selectively position a cursor at any desired location on display 1080. In addition, the cursor control device allows the user to select various commands, and input control signals. The cursor control device includes a number of signal generation devices for input control signals to system. Typically, a cursor control device may be a mouse, the button of the mouse being used to generate the signals. Alternatively or additionally, the client computer system may comprise a sensitive pad, and/or a sensitive screen.

The computer program may comprise instructions executable by a computer, the instructions comprising means for causing the above system to perform the method. The program may be recordable on any data storage medium, including the memory of the system. The program may for example be implemented in digital electronic circuitry, or in computer hardware, firmware, software, or in combinations of them. The program may be implemented as an apparatus, for example a product tangibly embodied in a machine-readable storage device for execution by a programmable processor. Method steps may be performed by a programmable processor executing a program of instructions to perform functions of the method by operating on input data and generating output. The processor may thus be programmable and coupled to receive data and instructions from, and to transmit data and instructions to, a data storage system, at least one input device, and at least one output device. The application program may be implemented in a high-level procedural or object-oriented programming language, or in assembly or machine language if desired. In any case, the language may be a compiled or interpreted language. The program may be a full installation program or an update program. Application of the program on the system results in any case in instructions for performing the method. The computer program may alternatively be stored and executed on a server of a cloud computing environment, the server being in communication across a network with one or more clients. In such a case a processing unit executes the instructions comprised by the program, thereby causing the method to be performed on the cloud computing environment.

## Claims

1. A computer implemented method for computing a score representing a percentage of occurrence of a clinical event in a period of time, the method comprising:
• obtaining (S10) a database including:
o a list of P characteristics within a vector *X*, associated with the clinical event, each characteristic (*p,* for p *=* {1*,* ···*, P*}) having a respective set of modalities (*pⱼ* for *j* = {1,··· , *nₚ*}, with *nₚ* the number of modalities of the characteristic p), and
o for each characteristic (*p*) of the list, a set of modality weights, each modality weight (*α*(*pⱼ* )) being associated with a respective modality (*j*) of the characteristic (*p*) and quantifying its effect on the percentage of occurrence of the clinical event;
• obtaining (S20), for a given patient, the modalities corresponding to a subset of the characteristics in the list (*p* ∈ *X**, wherein X* ⊂ *X*);
• extracting (S30), from the database, the modality weight associated with each modality obtained for the patient; and
• computing (S41, S42):
o the score (*scoreₚₐₜᵢₑₙₜ*) for the given patient by summing the extracted modalities weights, using the following formula: ${score}_{patient} = {\sum }_{{p}_{j} ∈ X *} α \left({p}_{j}\right) ;$ and
o a dispersion range associated with the computed score.

2. The method of claim 1, wherein the dispersion range comprises a maximum value (*Iₘₐₓ*) and a minimum value (*Iₘᵢₙ*), the list of characteristics (*p ∈ X*) including at least one remaining characteristic (*p* ∉ *X**) excluded from the subset of characteristics (*X**) obtained for the patient, the computing of the dispersion range comprising:
• computing (S43) the maximum value (*Iₘₐₓ*) by adding to the score the highest modality weight (*α*(*p⁺*)) of each remaining characteristic *p ∉ X**, using the following formula: ${I}_{max} = {score}_{patient} + {\sum }_{p ∉ X *} α \left({p}^{+}\right) ;$ and/or
• computing (S44) the minimum value (*Iₘᵢₙ*) by adding to the score the lowest modality weight (*α*(*p*⁻)) of each remaining characteristic *p ∉ X*,* using the following formula: ${I}_{min} = {score}_{patient} + {\sum }_{p ∉ X *} α \left({p}^{-}\right) .$

3. The method of claim 1 or 2, further comprising scaling (S50) the score and/or the dispersion range by applying a min-max scaler between a first value (e.g., 0) and a second value higher than the first value (e.g., 1).

4. The method of any of claims 1 to 3, further comprising displaying (S60) a first graphical representation representing the score and the dispersion range on a scale ranging from the first value (0%) to the second value (100%).

5. The method of claim 4, wherein the scale on which the score is displayed is subdivided into a low risk area, an intermediate risk area, and a high risk area, boundaries between the low risk area, the intermediate risk area and the high risk area corresponding to 0.33 and 0.66 quantiles of retrospective data.

6. The method of any of claims 1 to 5, further comprising displaying (S70) a second graphical representation indicating a relative contribution of each characteristic of the subset on the score computed for the patient.

7. The method of claim 6, further comprising computing (S80) the relative contribution of each characteristic, the computing of the relative contribution of a given characteristic *p* comprising:
• computing a contribution of the characteristic *p* using the following formula: 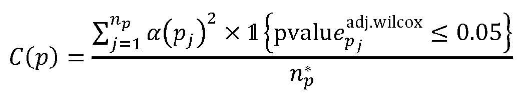 wherein:
o P is the total number of characteristics;
o *nₚ* is the number of modalities associated with the characteristic *p* (1 ≤ p ≤ P);
o ${n}_{p}^{∗}$ is the number of significant modalities associated to the characteristic p, with 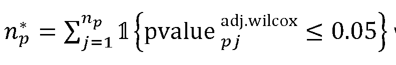 with 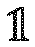 the indicator function;
∘ *α*(*pⱼ* ) is the modality weight associated with the modality *j* of the characteristic p (1 ≤ *p* ≤ *P*, 1 ≤ *j ≤ nₚ*); and
• computing the relative contribution of the characteristic p using the following formula: $RC \left(p\right) = \frac{C \left(p\right)}{{\sum }_{i = 1}^{P} C \left(i\right)}$

8. The method of claim 6 or 7, wherein the second graphical representation further indicates an impact of each modality obtained for the patient on the computed score, for example by using a gradient color.

9. The method of any of claims 1 to 8, wherein the set of modality weights of each characteristic are determined by interpreting a model modeling a multi-state process of a disease progression comprising states and transitions between the states, the clinical event corresponding to a given transition of the multi-state process.

10. The method of claim 9, wherein the model is a Cox model, the modality weights corresponding to the coefficients of the Cox model.

11. The method of claim 9, wherein the model is a deep learning neural network, each modality weight associated with a modality representing an average impact of the modality on transition probabilities computed by the model over a population of patients.

12. The method of claim 10 or 11, further comprising determining (S90) significant modalities of each characteristic by:
• obtaining a p-value for each modality weight, for example by applying a Wilcoxon test; and
• filtering the obtained p-values using a threshold.

13. A computer program comprising instructions which, when executed by a computer, cause the computer to carry out the method of any of claims 1-12.

14. A computer readable storage medium having recorded thereon a computer program of claim 13.

15. A system comprising a processor coupled to a memory and a graphical user interface, the memory having recorded thereon the computer program of claim 13.
